**Europäisches Patentamt**

**European Patent Office**

**Office européen des brevets**

(19)

(11) Veröffentlichungsnummer : **0 098 419**
**B1**

(12)
# EUROPÄISCHE PATENTSCHRIFT

(45) Veröffentlichungstag der Patentschrift :
**16.04.86**

(21) Anmeldenummer : **83105828.4**

(22) Anmeldetag : **14.06.83**

(51) Int. Cl.⁴ : **A 61 L 2/24**, A 61 L 2/26,
A 61 L 2/18, F 04 B 13/02,
G 05 D 11/00, G 01 F 11/02

(54) **Vorrichtung zum Zumischen von Desinfektionsmittel zu Wasser.**

(30) Priorität : **05.07.82 DE 3225076**

(43) Veröffentlichungstag der Anmeldung :
**18.01.84 Patentblatt 84/03**

(45) Bekanntmachung des Hinweises auf die Patenterteilung : **16.04.86 Patentblatt 86/16**

(84) Benannte Vertragsstaaten :
**AT BE CH DE FR GB IT LI LU NL SE**

(56) Entgegenhaltungen :
FR-A- 2 119 032
FR-A- 2 169 992
FR-A- 2 240 439

(73) Patentinhaber : **Bode Chemie GmbH & Co.**
**Melanchthonstrasse 27**
**D-2000 Hamburg 54 (DE)**

(72) Erfinder : **Göldner, H.**
**Berliner Ring 173 b**
**D-3070 Nienburg (DE)**

(74) Vertreter : **Haft, Berngruber, Czybulka**
**Hans-Sachs-Strasse 5**
**D-8000 München 5 (DE)**

Jouve, 18, rue St-Denis, 75001 Paris, France

## Beschreibung

Die Erfindung bezieht sich auf eine Vorrichtung zum Zumischen von Desinfektionsmittel zu Wasser gemäß dem Oberbegriff des Patentanspruches 1.

In Krankenhäusern muß ständig eine große Menge von Desinfektionsmittellösung mit bestimmter Konzentration zur Verfügung stehen. In größeren Krankenhäusern ist hierfür oftmals ein eigenes Leitungsnetz mit mehreren Zapfstellen für diese Desinfektionsmittellösung vorgesehen. Zu beachten ist hierbei, daß die Konzentration der Desinfektionsmittellösung exakt eingehalten wird.

Zur Herstellung der Desinfektionsmittellösungen wird z. B. Desinfektionsmittelkonzentrat mit einem Injektorverfahren bzw. mittels Pumpen dem Wasser zugemischt. Beim Injektorverfahren wird das Wasser über eine Düse in einen Mischraum eingestrahlt, in den eine Leitung aus einem Konzentratbehälter mündet. Abhängig vom Wasserdruck wird selbstregelnd eine entsprechende Menge Desinfektionsmittelkonzentrat angesaugt.

Bei der Zumischung über Pumpen ist die Konzentratförderung ebenfalls auf die Wasserförderungsmenge abgestellt. Ändert sich, etwa durch Korrosions- oder Alterserscheinungen die Wasserförderungsmenge, so bleibt die Konzentratmenge entweder konstant oder ändert sich zumindest nicht in diesem Rahmen. Insbesondere nachteilig ist dies, wenn für Wasserförderung und Konzentratförderung zwei unabhängige Pumpen mit entsprechend justierten Fördermengen verwendet werden.

Der Erfindung liegt die Aufgabe zugrunde, eine Vorrichtung der infrage stehenden Art so auszubilden, daß die Konzentration der erzeugten Desinfektionsmittellösung ständig konstant gehalten wird.

Diese Aufgabe ist gemäß der Erfindung durch die im kennzeichnenden Teil des Patentanspruches 1 angegebenen Merkmale gelöst.

Gemäß diesen Merkmalen werden die Wasserpumpe und die Konzentratpumpe jeweils als Kolbenpumpen ausgebildet, die miteinander zwangsgekoppelt sind. Vorzugsweise sind dabei die Pumpen Differentialkolbenpumpen. Die Volumina der Arbeitsräume der beiden Pumpen sind so aufeinander abgestimmt, daß das Verhältnis von Wassermenge und Konzentratmenge einen vorgegebenen Rahmen einhält. Das tatsächliche Mengenverhältnis wird dann durch die jeweiligen Kolbenwege bestimmt. Dieses Mengen- bzw. Mischungsverhältnis ist zusätzlich noch dadurch veränderbar, daß der Anlenkpunkt für die Kolbenstange der Konzentratpumpe variiert werden kann. Durch die Koppelung der beiden Kolbenstangen von Wasserpumpe und Konzentratpumpe ist dann das Mischungsverhältnis von Wasser und Konzentrat eindeutig festgelegt. Durch eine Vorrichtung gemäß der Erfindung bleibt auch der Wasserdruck konstant. Außerdem können zur Überprüfung des Mischungsverhältnisses an geeigneter Stelle der Vorrichtung, z. B. im Bereich des Mischraumes, Abnahmeleitungen vorgesehen werden, die zu einer geeigneten Kontrolleinrichtung führen.

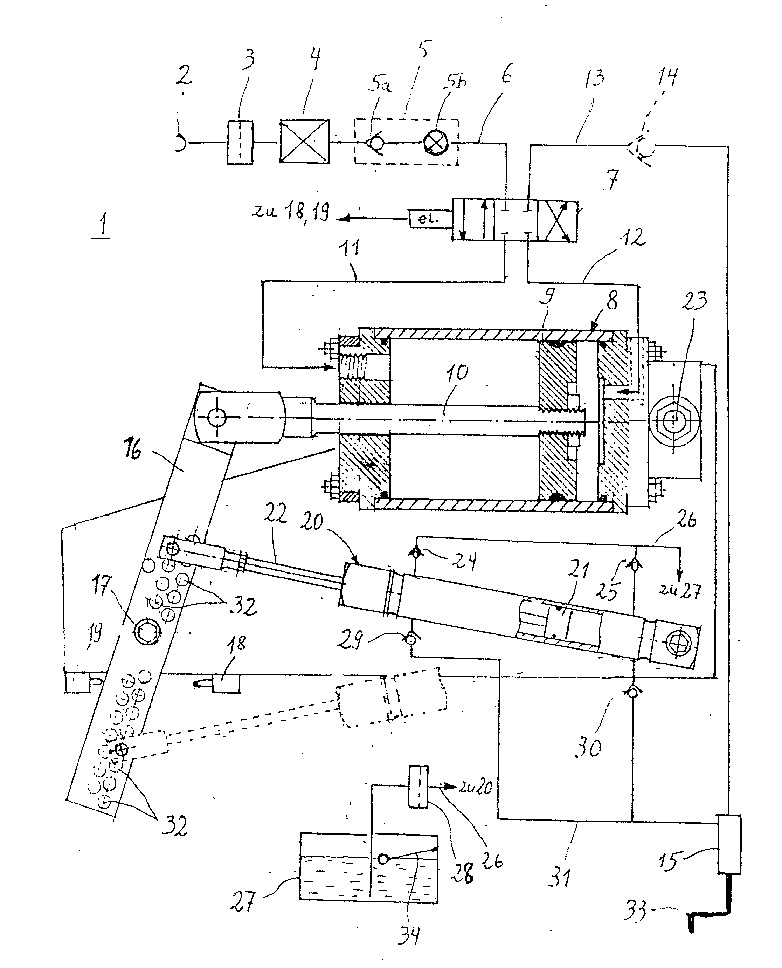

Weitere Ausgestaltungen und Vorteile der Erfindung gehen aus den Unteransprüchen in Verbindung mit der nachfolgenden Beschreibung hervor, in der anhand der einzigen Figur eine Vorrichtung zum Zumischen von Desinfektionsmittel zu Wasser näher erläutert ist.

Eine in der Figur gezeigte Vorrichtung 1 zum Zumischen von Desinfektionsmittelkonzentrat zu Wasser ist in einem hier nicht gezeigten Gehäuse untergebracht. Über einen Wasseranschluß 2 ist die Vorrichtung mit dem Wasserleitungsnetz verbunden. Das über den Wasseranschluß 2 gelieferte Frischwasser wird über einen Schmutzfänger 3 und eine übliche Wassermangelsicherung 4 einem Absperrventil 5 zugeleitet, welches ein Rückschlagventil 5a und ein übliches Kugelventil 5b mit Bedienknopf aufweist. Von diesem Absperrventil 5 führt eine Frischwasserzuleitung 6 zu einem elektrisch betätigten Steuerventil 7, in diesem Fall einem Vier/Zwei-Schieber. Mit diesem Schieber 7 werden die Bewegungen eines Arbeitszylinders 8 für das Frischwasser gesteuert, welcher als Differentialkolbenpumpe mit einem Kolben 9 und einer einseitigen Kolbenstange 10 ausgebildet ist. In die zu beiden Seiten des Kolbens 9 befindlichen Arbeitsräume des Arbeitszylinders 8 münden vom Schieber 7 ausgehende Leitungen 11 bzw. 12. Vom Schieber zweigt weiter eine Frischwasserableitung 13 ab, in der ein Rückschlagventil 14 vorgesehen ist, und die zu einem Mischraum, z. B. einem Mischrohr 15 führt.

Die Kolbenstange 10 des Arbeitszylinders 8 ist an einem Ende eines Schwenkhebels 16 angelenkt, der um einen Drehpunkt 17 verschwenkbar ist. Dem Schwenkhebel 16 sind zwei elektrische Endumschalter 18 und 19 zugeordnet, die das elektrische Stellglied des Schiebers 7 betätigen.

Der Schieber weist in diesem Falle drei Stellungen auf; die gezeigte Stellung ist die Absperrstellung. Diese Stellung ist an sich nicht notwendig, kann jedoch einer Ruhestellung der Endumschalter 18 und 19 zugeordnet werden. Wird das Absperrventil 5 geöffnet, so strömt in der ersten Stellung des Schiebers 7 über die Frischwasserzuleitung 6 und die Leitung 11 Frischwasser in den kolbenstangenseitigen Arbeitsraum des Arbeitszylinders 8, wodurch der Kolben 9 in der Figur nach rechtsgedrückt wird. Der Kolben verdrängt dabei das in dem anderen Arbeitsraum des Arbeitszylinders vorhandene Frischwasser über die Leitung 12, den Schieber 7 und die Ableitung 13 in das Mischrohr 15. Am Ende dieser Bewegung berührt der Schwenkhebel 16 den Endumschalter 19, so daß der Schieber in die andere Arbeitsposition überführt wird. In dieser Stellung strömt Frischwasser aus der Leitung 6 über den Schieber 7 und die Leitung 12 in den rechtsseitigen Arbeitsraum des Arbeitszylinders, während das in dem kolbenstangenseitigen Arbeitsraum vorhandene Frischwasser über die Leitung 11, den Schieber 7 und die Ableitung 13 in das Mischrohr 15 geleitet wird. Am Ende der Kolbenbewegung berührt der Schwenkhebel 16 den Endumschalter 18, so daß der Schieber 7 erneut in die vorherige Stellung überführt wird. Der geschilderte Arbeitszyklus wiederholt sich kontinuierlich, solange das Absperrventil geöffnet ist.

Die Vorrichtung weist außerdem eine Konzentratpumpe 20 auf, die ebenfalls als Arbeitszylinder in Form einer Differentialkolbenpumpe mit einem Arbeitskolben 21 und einer einseitigen Kolbenstange 22 ausgebildet ist. Die Konzentratpumpe 20 ist an dem der Kolbenstange 22 abgewandten Ende an einem Bolzen 23 schwenkbar gelagert. Die Kolbenstange 22 ist an dem Schwenkhebel 16 angelenkt. Die beiden Arbeitsräume der Konzentratpumpe 20 sind über Ansaugrückschlagventile 24 und 25 und eine Ansaugleitung 26 mit einem Konzentratbehälter 27 verbunden. In der Ansaugleitung 26 ist noch ein Schmutzfänger 28 vorgesehen. Ferner sind die beiden Arbeitsräume der Konzentratpumpe 20 über Abflußrückschlagventile 29 und 30 und eine Abflußleitung 31 mit dem Mischrohr 15 verbunden.

Die Hebelarme für die beiden Kolbenstangen 10 bzw. 22 von Wasserpumpe 8 und Konzentratpumpe 20, d. h. der jeweilige Abstand zwischen dem Drehpunkt 17 des Schwenkhebels 16 und dem Anlenkpunkt der jeweiligen Kolbenstange an dem Schwenkhebel 16 bestimmen in Verbindung mit dem Verhältnis der Volumina der beiden Pumpen 8 bzw. 20 das Mischungsverhältnis zwischen Frischwasser und Desinfektionsmittelkonzentrat. Zur Einstellung dieses Mischungsverhältnisses kann die Kolbenstange 22 der Konzentratpumpe 20 an unterschiedlichen Stellen des Schwenkhebels angelenkt werden, was in der Figur durch mehrere Stellöcher 32 im Schwenkhebel angedeutet ist. Soll das Mischungsverhältnis geändert werden, so wird die Kolbenstange entsprechend in einem anderen Stelloch befestigt. Die Konzentratpumpe 20 kann dieser Verstellung folgen, da sie am Bolzen 23 schwenkbar gelagert ist.

Bei der oben geschilderten Bewegung des Kolbens 9 der Wasserpumpe 8 in der Figur nach rechts wird entsprechend der Arbeitskolben 21 der Konzentratpumpe 20 ebenfalls nach rechts bewegt. Bei dieser Bewegung wird einmal aus dem Konzentratbehälter 27 über das Rückschlagventil 24 Desinfektionsmittelkonzentrat in den kolbenstangenseitigen Arbeitsraum der Konzentratpumpe gesaugt und zum anderen Desinfektionsmittelkonzentrat über das Abflußrückschlagventil 30 mit der Leitung 31 dem Mischrohr zugeführt. Erst in dem Mischrohr werden Frischwasser und Konzentrat miteinander vermischt und ergeben eine Desinfektionsmittellösung mit dem gewünschten Mischungsverhältnis. Bei der gegensinnigen Kolbenbewegung wird in den rechtsseitigen Kolbenraum über die Ansaugleitung 26 und das Ansaugrückschlagventil 27 Desinfektionsmittelkonzentrat in den rechtsseitigen Arbeitsraum der Konzentratpumpe gesaugt; gleichzeitig wird aus dem kolbenstangenseitigen Arbeitsraum der Konzentratpumpe 20 über das andere Abflußrückschlagventil 29 und die Abflußleitung 31 Konzentrat dem Mischrohr 15 zugeführt. Dieser Arbeitszyklus wiederholt sich entsprechend, wie oben geschildert.

Durch die Zwangskopplung der beiden Pumpen 8 bzw. 20 über den Schwenkhebel 16 wird das einmal eingestellte Mischungsverhältnis immer eingehalten. Das Frischwasser sowie das Desinfektionsmittelkonzentrat fließen getrennt zu dem Mischrohr 15, das unmittelbar vor einem Auslauf, z. B. einer üblichen Zapfstelle 33 angeordnet ist. Dadurch ist gewährleistet, daß bei jeder Entnahme die Desinfektionsmittellösung frisch zugemischt wird. Eine chemische Reaktion zwischen Produkt und den einzelnen Steuerelementen wird so vermieden und die Lösung durch Restbestände im Gerät nicht abgebaut. Ein Vorteil dieser Art Zumischung liegt auch darin, daß das Frischwasser vom Wassernetz getrennt und erst direkt bei der Entnahme an der Zapfstelle 33 mit Konzentrat vermischt wird. Der Kolben 9 des Arbeitszylinders bzw. der Wasserpumpe 8 bewirkt auf diese Weise eine eindeutige Systemtrennung. Eine Verbindung von Desinfektionsmittelkonzentrat zum Frischwassernetz wird dadurch grundsätzlich vermieden.

In dem Konzentratbehälter 27 ist ferner ein hier nur angedeuteter Schwimmer 34 mit einem Annäherungsschalter vorgesehen, der den Flüssigkeitsstand in dem Konzentratbehälter 27 überwacht. Der Schwimmer mit dem Annäherungsschalter ist so eingestellt, daß die in dem Konzentratbehälter 27 eintauchende Ansaugleitung 26 auch bei niedrigstem Konzentratstand noch in das Desinfektionsmittelkonzentrat eintaucht. Hierdurch wird verhindert, daß Luft angesaugt wird und sich in dem System ein Luftpolster bildet. Dieses würde bedeuten, daß z. B. die nächsten 10 bis 20 Liter Desinfektionsmittellösung nach Auswechseln des Konzentratbehälters 27 unterdosiert sein könnten. Eine solche Möglichkeit wird durch die Konzentratüberwachung ausgeschaltet.

Die Entnahme von fertiger Desinfektionsmittellösung mit der beschriebenen Vorrichtung erfolgt ohne Motorkraft und elektrischem Antrieb; das System arbeitet noch bei geringen Wasserdrücken von z. B. 0,8 bar. Aus steuertechnischen Gründen wird jedoch über die Wassermangelsicherung eine Abschaltung der Vorrichtung bereits bei 1 bar Wasserdruck vorgesehen.

Die beschriebene Vorrichtung kann auch zur Versorgung von mehreren Zapfstellen betrieben werden, d. h. als zentrale Versorgungseinrichtung für ein gesamtes Desinfektionsmittellösungs-Leitungsnetz mit mehreren Fernzapfstellen. Anstelle der Zapfstellen können auch Sprühlanzen angeschlossen werden. Im Ruhezustand ist die gesamte Vorrichtung drucklos.

Selbstverständlich kann auch, wie in der Figur gestrichelt angedeutet, die Kolbenstange der Konzentratpumpe am Schwenkhebel in bezug auf den Anlenkpunkt der Wasserpumpe jenseits des Schwenkhebeldrehpunktes angelenkt sein. Die Kolbenbewegungen von Wasserpumpe und Konzentratpumpe verlaufen dann gegensinnig.

**Patentansprüche**

1. Vorrichtung zum Zumischen von Desinfektionsmittel zu Wasser in einem bestimmten Mischungsverhältnis mit einer an ein Wasserleitungsnetz angeschlossenen Wasserpumpe, einer Desinfektionsmittelkonzentratpumpe, die aus einem Konzentratbehälter Desinfektionsmittelkonzentrat ansaugt, sowie einem Mischraum, dadurch gekennzeichnet, daß Wasserpumpe (8) und Konzentratpumpe (20) als Kolbenpumpen mit beidseitig beaufschlagbaren Kolben ausgebildet sind, daß die beiden Arbeitsräume der Wasserpumpe über Steuerventile (Steuerschieber 7) mit dem Wasserleitungsnetz und mit dem Mischraum (Mischrohr 15) verbunden sind, daß die Arbeitsräume der Konzentratpumpe (20) über Ansaugrückschlagventile (24, 25) mit dem Konzentratbehälter (27) und über Abflußrückschlagventile (29, 30) mit dem Mischraum (15) verbunden sind, daß die Kolbenstangen (10, 22) der beiden Kolbenpumpen (8, 20) mit einen, an einem Drehpunkt (17) angelenkten Schwenkhebel (16) an unterschiedlichen Hebelpunkten drehbar befestigt sind, und daß zur Steuerung der Kolbenstangenbewegungen für den Schwenkhebel (16) die Steuerventile (Schieber 7) der Wasserpumpe (8) betätigende Endumschalter (18, 19) vorgesehen sind.

2. Vorrichtung nach Anspruch 1, dadurch gekennzeichnet, daß Wasserpumpe (8) und Konzentratpumpe (20) als Differentialkolbenpumpen ausgebildet sind.

3. Vorrichtung nach einem der Ansprüche 1 und 2, dadurch gekennzeichnet, daß die Steuerventile für die Wasserpumpe (8) in einem elektrisch betätigbaren Vier/Zwei-Schieber (7) zusammengefaßt sind.

4. Vorrichtung nach einem der vorhergehenden Ansprüche, dadurch gekennzeichnet, daß in der zum Mischraum (15) führenden Abflußleitung (13) der Wasserpumpe (8) ein Rückschlagventil (14) vorgesehen ist.

5. Vorrichtung nach einem der vorhergehenden Ansprüche, dadurch gekennzeichnet, daß in der zu der Wasserpumpe (8) führenden Frischwasserzuleitung (6) eine Wassermangelsicherung (4) vorgesehen ist.

6. Vorrichtung nach einem der vorhergehenden Ansprüche, dadurch gekennzeichnet, daß in der zu der Wasserpumpe (8) führenden Frischwasserzuleitung (6) ein Absperrventil (5) mit einem Rückschlagventil (5a) vorgesehen ist.

7. Vorrichtung nach einem der vorhergehenden Ansprüche, dadurch gekennzeichnet, daß der Schwenkhebel (16) zur Variierung des Anlenkpunktes der Kolbenstange (22) der Konzentratpumpe (20) mehrere Stellöcher (32) aufweist.

8. Vorrichtung nach Anspruch 7, dadurch gekennzeichnet, daß die Konzentratpumpe (20) an ihrem der Kolbenstange (22) abgewandten Ende schwenkbar (bei Bolzen 23) angelenkt ist.

**Claims**

1. An equipment for the addition of a disinfectant to water at a specific mixing ratio, comprising a water pump connected to a water supply system, a disinfectant concentrate pump taking in disinfectant concentrate from a concentrate receptacle, and a mixing chamber, characterized in that the water pump (8) and the concentrate pump (20) are constructed as piston pumps with pistons which are adapted to be acted upon on two sides, that the two operating spaces of the water pump are connected via control valves (control slide valves 7) to the water supply system and to the mixing chamber (mixing tube 15), that the operating spaces of the concentrate pump (20) are connected to the concentrate receptacle (27) via non-return intake valves (24, 25) and to the mixing chamber (15) via non-return discharge valves (29, 30), that the piston rods (10, 22) of the two piston pumps (8, 20) are rotatably secured to different fulcrums by means of a pivot lever (16) pivotally connected to a centre of motion (17), and that for controlling the piston rod movements there are provided travel reversing switches (18, 19) for the pivot lever (16), said travel reversing switches (18, 19) actuating the control valves (control slide valves 7) of the water pump (8).

2. An equipment according to claim 1, characterized in that the water pump (8) and the concentrate pump (20) are constructed as differential piston pumps.

3. An equipment according to one of the claims 1 and 2, characterized in that the control valves for the water pump (8) are combined to form an electrically actuable four/two slide valve (7).

4. An equipment according to one of the preceding claims, characterized in that a non-return valve (14) is provided in the discharge conduit (13) of the water pump (8) leading to the mixing chamber (15).

5. An equipment according to one of the preceding claims, characterized in that a water shortage prevention means (4) is provided in the fresh water supply conduit (6) leading to the water pump (8).

6. An equipment according to one of the preceding claims, characterized in that a shut-off valve (5) including a non-return valve (5a) is provided in the fresh water supply conduit (6) leading to the water pump (8).

7. An equipment according to one of the preceding claims, characterized in that the pivot lever (16) is provided with a plurality of adjustment holes (32) for varying the articulation point of the piston rod (22) of the concentrate pump (20).

8. An equipment according to claim 7, characterized in that the concentrate pump (20) is pivotally connected (at pin 23) at its end facing away from the piston rod (22).

## Revendications

1. Dispositif permettant le dosage d'un produit de désinfection dans l'eau selon un rapport déterminé, comprenant une pompe à eau raccordée à un réseau de distribution d'eau, une pompe à produit de désinfection concentré qui aspire le produit de désinfection concentré contenu dans un réservoir de produit concentré, ainsi qu'une chambre de mélange, caractérisé en ce que la pompe à eau (8) et la pompe à produit concentré (20) sont des pompes à piston avec un piston sollicité des deux côtés, en ce que les deux espaces de travail de la pompe à eau sont reliés par des vannes de distribution (tiroirs de distribution 7) au réseau de distribution d'eau et à la chambre de mélange (tuyau de mélange 15), en ce que les espaces de travail de la pompe à produit concentré (20) sont reliés au réservoir de produit concentré (27) par des vannes de retenue d'aspiration (24, 25) et à la chambre de mélange par des vannes de retenue d'écoulement (29, 30), en ce que les tiges de piston (10, 22) des deux pompes à piston (8, 20) sont fixées de façon pivotante sur un levier de pivotement (16) articulé sur un pivot (17) sur différents points d'appui de levier, et en ce qu'il est prévu des inverseurs de fin de course (18, 19) commandant les vannes de distribution (tiroir 7) de la pompe à eau (8) pour le réglage du déplacement des tiges de piston pour le levier de pivotement (16).

2. Dispositif selon la revendication 1, caractérisé en ce que la pompe à eau (8) et la pompe à produit concentré (20) sont des pompes à piston différentiel.

3. Dispositif selon l'une des revendications 1 et 2, caractérisé en ce que les vannes de distribution de la pompe à eau (8) sont rassemblées dans un tiroir (7) à deux ou quatre passages commandable électriquement.

4. Dispositif selon l'une des revendications précédentes, caractérisé en ce qu'il est prévu, dans la conduite d'écoulement (13) de la pompe à eau (8) conduisant à la chambre de mélange (15) un clapet de non-retour (14).

5. Dispositif selon l'une des revendications précédentes, caractérisé en ce qu'il est prévu, dans la conduite d'eau fraîche (6) conduisant à la pompe à eau (8), une sécurité (4) contre le manque d'eau.

6. Dispositif selon l'une des revendications précédentes, caractérisé en ce qu'il est prévu, dans la conduite d'eau fraîche (6) conduisant à la pompe à eau (8), une vanne d'arrêt (5) avec un clapet de non-retour (5a).

7. Dispositif selon l'une des revendications précédentes, caractérisé en ce que le levier de pivotement (16) destiné à faire varier le point d'articulation de la tige de piston (22) de la pompe à produit concentré (20) présente plusieurs trous de réglage (32).

8. Dispositif selon la revendication 7, caractérisé en ce que la pompe à produit concentré (20) est articulée de façon pivotante (par la cheville 23) à son extrémité opposée à la tige de piston (22).